# EUROPEAN PATENT SPECIFICATION

(11) **EP 2 593 162 B1**
(45) Date of publication and mention of the grant of the patent: **03.06.2015**
(21) Application number: 11725475.5
(22) Date of filing: 17.06.2011
(51) Int. Cl.: A61M 5/315, A61M 5/28, A61M 5/24

(54) **A piston rod foot**
Kolbenstangenfuß
Pied de tige de piston

(30) Priority: 19.07.2010 US 365542 P; 13.07.2010 EP 10169406
(43) Date of publication of application: 22.05.2013
(73) Proprietor: Novo Nordisk A/S, 2880 Bagsværd (DK)
(72) Inventor: HEMMINGSEN, Hans, DK-2880 Bagsværd (DK)
(86) International application number: PCT/EP2011/060109
(87) International publication number: WO 2012/007246

(56) References cited:
- EP-A1- 2 174 681
- DE-A1-102009 052 666
- US-A- 5 181 918

## Description

### THE TECHNICAL FIELD OF THE INVENTION:

The invention relates to an injection pen in which a piston rod moves a piston or plunger forward inside a cartridge and more specifically the invention relates to a piston rod foot or washer for such injection pen.

### DESCRIPTION OF RELATED ART:

People suffering from diabetes often have to inject themselves with insulin at a daily basis. For this purpose a great number of different pen systems have been developed over the last 30 years. Common for pen injectors is that they contain a container or cartridge containing the liquid drug to be injected. The cartridge is provided with a piston which is moved forward in order to transfer the liquid drug from the injection pen and into the body of the user.

An example of such commercial successful injection pen, the Flexpen® by Novo Nordisk A/S, is given in US 6,235,004. The cartridge (89) as e.g. depictured in figure 15-17 contains the liquid drug to be expelled. At the proximal end the cartridge (89) is closed by a rubber piston which is moved forward inside the cartridge (89) by a piston rod (7). In order to transfer and distribute the force from the piston rod (7) to the rubber piston, a piston rod foot (9) is provided between the piston rod (7) and the rubber piston.

For prefilled injection pens which is characterized by the fact that they are discarded when the user has used the prefilled amount of drug there is no possibility for the user to return the piston rod. Often such injection pens are sealed such that the user can not physically obtain contact with the piston rod. In such injection pens the piston rod foot is normally laying between the rubber piston and the piston rod without being attached to any of the two components since this is the easiest way to assemble the injection pen. However, should the piston rod and/or the rubber piston for some reason move unprovoked in opposite directions the piston rod foot could tilt which would course the injection pen to malfunction

In some injection devices e.g. as disclosed in GB 2,117,249 and US 6,569,126, a one-way element is provided which secures that the rubber piston can only move in one direction. These one-way elements slides inside the cartridge and has an outside diameter slightly larger than the inside diameter of the cartridge. Another kind of one-way element is disclosed in US 5,135,512, which shows a washer having a diameter larger than the diameter of the syringe housing. However neither of these known syringes is provided with a piston rod foot.

### DESCRIPTION OF THE INVENTION:

It is an object of the present invention to provide an injection pen having a higher degree of safety. A particular object is to provide a piston rod foot that is more stable and which do not easily tilt. A further object is to provide a piston rod foot which besides the above also can be incorporated in an existing assembly process without having to change to assembly lines.

In a first embodiment, the piston rod foot or washer has a number of arms engaging the wall of the cartridge. The numbers can be one or more. If the movable piston or the piston rod is moved away from the piston rod foot this will provide a gap either between the piston rod foot and the movable piston or between the piston rod foot and the piston rod or in some cases both. Such a gap will inform the user that something is not as it should be, however when the user primes the injection pen by performing one or more air shots, the piston rod foot will again be brought into engagement with both the movable piston and the piston rod. No matter what the distance is between the movable piston and the piston rod it is now secured that the piston root foot will not tilt as it is secured against the inner wall of the cartridge.

The piston rod foot preferably has identical upper and bottom side such that it can be placed in either direction in the injection pen. Since the only difference to an existing piston rod foot is geometrical the new and improved piston rod foot can easily be implemented in an existing production line. The piston rod foot has in one embodiment an outside diameter smaller than the inner wall of the cartridge whereby an easy mounting in the cartridge is secured.

The imaginative diameter formed by the outer ends of the plurality of arms can in one embodiment be substantial identical the inside diameter of the cartridge in which case the arms are guided by, and on, the inside surface of the cartridge. In another embodiment the arms, or rather the tangential circle of the arms can be slightly larger than the inside diameter of the cartridge in order to provide a frictional engagement with the inner wall of the cartridge.

Further, the width of the arms provides security against tilt, and this width is preferably the same as the width of the piston rod foot itself. The arms can be either stiff or resilient. When the imaginative, tangential circle of the arms are lager than the inside diameter of the cartridge, the arms are preferably resilient as this will ease the mounting of the piston rod foot inside the cartridge.

### DEFINITIONS:

An "injection pen" is typically an injection apparatus having an oblong or elongated shape somewhat like a pen for writing. Although such pens usually have a tubular cross-section, they could easily have a different cross-section such as triangular, rectangular or square or any variation around these geometries.

As used herein, the term "drug" is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

Correspondingly, the term "subcutaneous" injection is meant to encompass any method of transcutaneous delivery to a subject.

"Cartridge" is the term used to describe the container containing the drug. Cartridges are usually made from glass but could also be moulded from any suitable polymer. A cartridge or ampoule is preferably sealed at one end by a pierceable membrane which can be pierced e.g. by an injection needle. The opposite end is closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. A "piston rod foot" is an element which distributes the force from the piston rod to the movable plunger or piston and is usually located between the piston rod and the movable piston or plunger.

All headings and sub-headings are used herein for convenience only and should not be constructed as limiting the invention in any way.

The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1: show the medical drug delivery apparatus according to the invention.
- Figure 2: show a top view of the piston rod foot inside the cartridge.
- Figure 3: show an example of a piston rod foot.
- Figure 4: show a different example of a piston rod foot.
- Figure 5: show a cross section of the piston rod foot depictured in figure 4.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as there relative dimensions are intended to serve illustrative purposes only.

In that context it may be convenient to define that the term "distal end" in the appended figures is meant to refer to the end pointing towards the needle cannula penetrating the patient whereas the term "proximal end" is meant to refer to the opposite end.

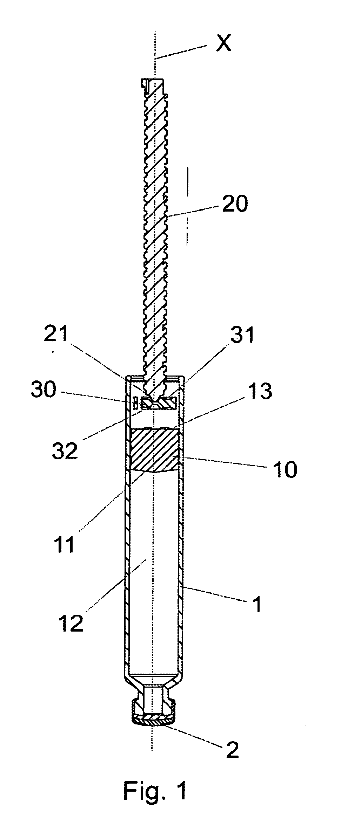

Figure 1 discloses a medical drug delivery apparatus which comprises of a cartridge 1, a rubber piston 10, a piston rod 20 and a piston rod foot 30.

The cartridge 1 is closed at its distal end sealed by a flexible membrane 2 which can be penetrated by a not shown injection needle. At the proximal end the cartridge 1 is closed by the movable rubber piston 10. The rubber piston 10 has a front wall 11 which is in contact with the drug encapsulated in the space 12 between the flexible membrane 2 and front wall 11 of the movable rubber piston 10.

The piston rod 20 is connected to a not shown injection mechanism which moves the piston rod 20 forward during injection. During injection, the distal end 21 of the piston rod 20 abuts the proximal surface 31 of the piston rod foot 30. The distal surface 32 of the piston rod foot 30 is in contact with the rubber piston 10 at its back wall 13 such that a force applied to the piston rod 20 is transmitted to the rubber piston 10 at a larger cross section.

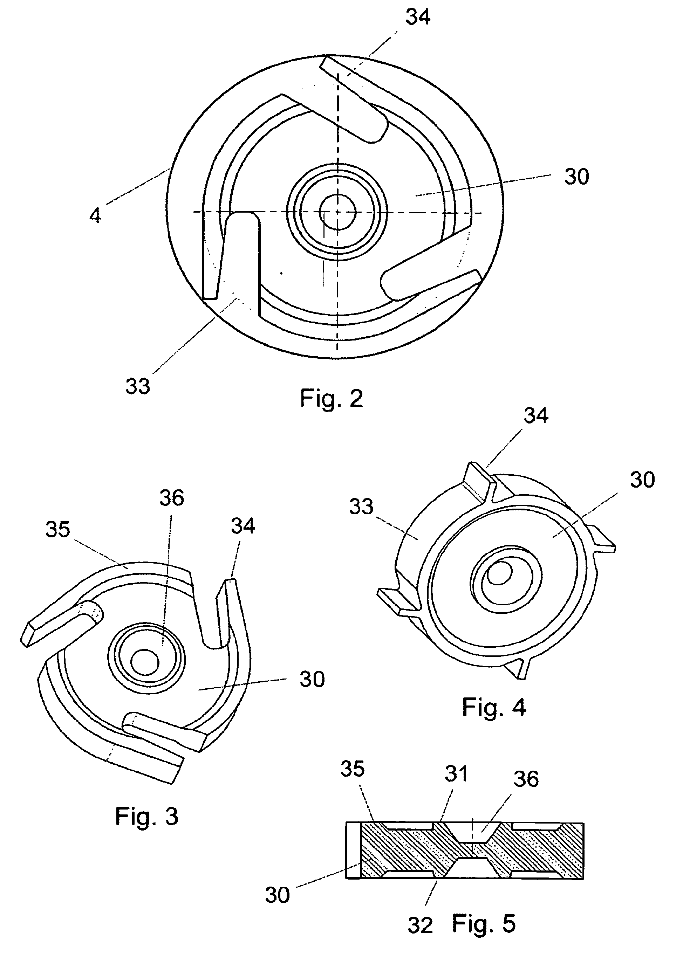

Figure 2 disclose a top view of the piston rod foot 30 inside the cartridge 1. The outside surface 33 of the piston rod foot 30 has a diameter as indicated with a dotted line which is somewhat smaller than the diameter of the inner wall 4 of the cartridge 1. The piston rod foot 30 is provided with a plurality of arms 34 which stretches in a direction perpendicular to the longitudinal axis X of the cartridge 1 and which arms 34 engages the inner wall 4 of the cartridge 1 thereby creating friction between the inner wall 4 of the cartridge 1 and the piston rod foot 30.

Figure 3 and 4 discloses different examples of a piston rod foot 30. The piston rod foot 30 preferably has identical distal 32 and proximal 31 surfaces such that it can be mounted in the injection device with either surface pointing in the distal or proximal direction. A circumferential ridge 35 is provided such that the ridge 35 abuts the rubber piston 10 in the peripheral zone in order to apply the pressure as close to the inner wall 4 of the cartridge 1 as possible. At the centre the piston rod foot is provided with a circular depression 36 which is formed with conical walls to receive the distal end 21 of the piston rod 20.

Some preferred embodiments have been shown in the foregoing, but it should be stressed that the invention is not limited to these, but may be embodied in other ways within the subject matter defined in the following claims.

## Claims

1. A medical drug delivery apparatus for delivering a liquid drug, comprising
A cartridge (1) containing the liquid drug to be delivered, the cartridge (1) comprising a distal end and a proximal end connected by an inner wall (4) forming a space (12) containing the liquid drug, the distal end being closed by a flexible membrane (2), and the proximal end being closed by a movable piston (10) having a front-wall (11) and a back-wall (13), and which piston (10) can be moved inside the cartridge (1) by a piston rod (20) thereby decreasing the variable space (12) between the flexible membrane (2) and a front wall (11) of the piston (10), and
A piston rod foot (30) provided between the piston rod (20) and the piston (10), the piston rod foot (30) having a distal surface (32) for abutting the back-wall (13) of the piston (10) and a proximal surface (31) abutting the piston rod (20),
**Characterized in that**, the piston rod foot (30) has a number of arms (34) extending in a direction perpendicular to a longitudinal axis (X) of the cartridge (1) and which arms (34) engages the inner wall (4) of the cartridge (1).

2. A medical drug delivery apparatus according to claim 1, **characterized in that**, the piston rod foot (3) has an outer surface (33) with a diameter smaller than the diameter of the inner wall (4) of the cartridge (1).

3. A medical drug delivery apparatus according to claim 1 or 2, **characterized in that**, a peripheral circle drawn tangential to the arms (34) has a diameter substantial identical to the diameter of the inner wall (5) of the cartridge (1) thereby obtaining geometric guidance of the piston rod foot (30) against the inner wall of the cartridge (1).

4. A medical drug delivery apparatus according to 1 or 2, a peripheral circle drawn tangential to the arms (34) has a diameter larger than the diameter of the inner wall (4) of the cartridge (1).thereby generating a force of friction between the piston rod foot (30) and the inner wall (4) of the cartridge (1).

5. A medical drug delivery apparatus according to anyone of the preceding claims, **characterized in that**, the arms (34) have a width substantially equal to the width of the outer surface (33) of the piston rod foot (30).

6. A medical drug delivery apparatus according to anyone of the preceding claims, **characterized in that** the arms (34) are resilient.

## Patentansprüche

1. Medizinische Arzneimittelabgabevorrichtung für die Abgabe eines flüssigen Arzneimittels, umfassend
eine Kartusche (1), die das abzugebende flüssige Arzneimittel enthält, wobei die Kartusche (1) ein distales Ende und ein proximales Ende aufweist, die durch eine Innenwand (4), welche einen Raum (12) bildet, der das flüssige Arzneimittel enthält, verbunden sind, wobei das distale Ende durch eine elastische Membran (2) verschlossen ist und das proximale Ende durch einen beweglichen Kolben (10) mit einer Vorderwand (11) und einer Hinterwand (13) verschlossen ist, und wobei der Kolben (10) innerhalb der Kartusche (1) durch eine Kolbenstange (20) bewegt werden kann, wodurch der veränderliche Raum (12) zwischen der elastischen Membran (2) und einer Vorderwand (11) des Kolbens (10) verringert wird, und
einen Kolbenstangenfuß (30), der zwischen der Kolbenstange (20) und dem Kolben (10) bereitgestellt wird, wobei der Kolbenstangenfuß (30) eine distale Fläche (32) zum Anliegen an der Hinterwand (13) des Kolbens (10) und eine proximale Fläche (31) zum Anliegen an der Kolbenstange (20) aufweist,
**dadurch gekennzeichnet, dass** der Kolbenstangenfuß (30) eine Anzahl an Armen (34) aufweist, die sich in eine Richtung senkrecht zu einer Längsachse (X) der Kartusche (1) erstrecken und wobei die Arme (34) mit der Innenwand (4) der Kartusche (1) in Eingriff stehen.

2. Medizinische Arzneimittelabgabevorrichtung nach Anspruch 1, **dadurch gekennzeichnet, dass** der Kolbenstangenfuß (30) eine Außenfläche (33) mit einem Durchmesser aufweist, der kleiner ist als der Durchmesser der Innenwand (4) der Kartusche (1).

3. Medizinische Arzneimittelabgabevorrichtung nach Anspruch 1 oder 2, **dadurch gekennzeichnet, dass** ein Umfangskreis, der tangential zu den Armen (34) gezeichnet wird, einen Durchmesser aufweist, der im Wesentlichen zu dem Durchmesser der Innenwand (4) der Kartusche (1) identisch ist, wodurch eine geometrische Führung des Kolbenstangenfußes (30) gegen die Innenwand der Kartusche (1) erhalten wird.

4. Medizinische Arzneimittelabgabevorrichtung nach Anspruch 1 oder 2, wobei ein Umfangskreis, der tangential zu den Armen (34) gezeichnet wird, einen Durchmesser aufweist, der größer ist als der Durchmesser der Innenwand (4) der Kartusche (1), wodurch eine Reibungskraft zwischen dem Kolbenstangenfuß (30) und der Innenwand (4) der Kartusche (1) erzeugt wird.

5. Medizinische Arzneimittelabgabevorrichtung nach einem der vorhergehenden Ansprüche, **dadurch gekennzeichnet, dass** die Arme (34) eine Breite aufweisen, die im Wesentlichen gleich der Breite der Außenfläche (33) des Kolbenstangenfußes (30) ist.

6. Medizinische Arzneimittelabgabevorrichtung nach einem der vorhergehenden Ansprüche, **dadurch gekennzeichnet, dass** die Arme (34) elastisch sind.

## Revendications

1. Appareil médical pour l'administration de médicaments, pour l'administration d'un médicament liquide, comprenant :
une cartouche (1) contenant le médicament liquide à administrer, la cartouche (1) comprenant une extrémité distale et une extrémité proximale connectées par une paroi interne (4) formant un espace (12) contenant le médicament liquide,
l'extrémité distale étant fermée par une membrane flexible (2), et l'extrémité proximale étant fermée par un piston déplaçable (10) ayant une paroi avant (11) et une paroi arrière (13), lequel piston (10) pouvant être déplacé à l'intérieur de la cartouche (1) par une tige de piston (20) pour diminuer ainsi l'espace variable (12) entre la membrane flexible (2) et une paroi avant (11) du piston (10), et
un pied de tige de piston (30) disposé entre la tige de piston (20) et le piston (10), le pied de tige de piston (30) ayant une surface distale (32) destinée à buter contre la paroi arrière (13) du piston (10) et une surface proximale (31) butant contre la tige de piston (20),
**caractérisé en ce que** le pied de tige de piston (30) présente un certain nombre de bras (34) s'étendant dans une direction perpendiculaire à un axe longitudinal (X) de la cartouche (1), et lesquels bras (34) s'engagent avec la paroi interne (4) de la cartouche (1).

2. Appareil médical pour l'administration de médicaments selon la revendication 1, **caractérisé en ce que** le pied de tige de piston (30) a une surface externe (33) dont le diamètre est inférieur au diamètre de la paroi interne (4) de la cartouche (1).

3. Appareil médical pour l'administration de médicaments selon la revendication 1 ou 2, **caractérisé en ce qu'**un cercle périphérique tracé tangentiellement aux bras (34) présente un diamètre substantiellement identique au diamètre de la paroi interne (4) de la cartouche (1) pour obtenir ainsi un guidage géométrique du pied de tige de piston (30) contre la paroi interne de la cartouche (1).

4. Appareil médical pour l'administration de médicaments selon la revendication 1 ou 2, **caractérisé en ce qu'**un cercle périphérique tracé tangentiellement aux bras (34) présente un diamètre plus grand que le diamètre de la paroi interne (4) de la cartouche (1), pour générer ainsi une force de friction entre le pied de tige de piston (30) et la paroi interne (4) de la cartouche (1).

5. Appareil médical pour l'administration de médicaments selon l'une quelconque des revendications précédentes, **caractérisé en ce que** les bras (34) ont une largeur sensiblement égale à la largeur de la surface externe (33) du pied de tige de piston (30).

6. Appareil médical pour l'administration de médicaments selon l'une quelconque des revendications précédentes, **caractérisé en ce que** les bras (34) sont élastiques.
